# EUROPEAN PATENT SPECIFICATION

(11) **EP 2 717 768 B1**
(45) Date of publication and mention of the grant of the patent: **14.10.2015**
(21) Application number: 12728366.1
(22) Date of filing: 07.06.2012
(51) Int. Cl.: A61B 5/053, A61K 9/06, A61K 47/44, A61N 1/04

(54) **ELECTRODE SENSOR KIT AND ELECTRODE SENSOR ASSEMBLY FOR ESTABLISHING ELECTRICAL CONTACT WITH SKIN FOR ELECTRO-IMPEDANCE TOMOGRAPHY (EIT) IMAGING**
ELEKTRODENSENSORKIT UND ELEKTRODENSENSORANORDNUNG ZUR HERSTELLUNG EINES ELEKTRISCHEN KONTAKTS MIT DER HAUT ZUR ELEKTROIMPEDANZTOMOGRAPHIE-BILDGEBUNG
KIT DE CAPTEUR D'ÉLECTRODE ET ENSEMBLE DE CAPTEUR D'ÉLECTRODE DESTINÉS À ÉTABLIR UN CONTACT ÉLECTRIQUE AVEC LA PEAU POUR L'IMAGERIE TOMOGRAPHIQUE PAR IMPÉDANCE ÉLECTRIQUE (EIT)

(30) Priority: 07.06.2011 CH 959112011
(43) Date of publication of application: 16.04.2014
(73) Proprietor: Swisstom AG, 7302 Landquart (CH)
(72) Inventor: BRUNNER, Josef X., CH-7000 Chur (CH); BÖHM, Stephan, 21481 Lauenburg/Elbe (DE)
(74) Representative: Hasler, Erich
(86) International application number: PCT/CH2012/000126
(87) International publication number: WO 2013/113130

(56) References cited:
- WO-A1-97/05171
- WO-A2-2010/078441
- US-A- 3 567 657
- US-A1- 2005 136 077
- US-A1- 2007 060 862
- MCADAMS E T ET AL: "FACTORS AFFECTING ELECTRODE-GEL-SKIN INTERFACE IMPEDANCE IN ELECTRICAL IMPEDANCE TOMOGRAPHY", MEDICAL AND BIOLOGICAL ENGINEERING AND COMPUTING, SPRINGER, HEILDELBERG, DE, vol. 34, no. 6, 1 November 1996 (1996-11-01), pages 397-408, XP000636563, ISSN: 0140-0118

## Description

### TECHNICAL FIELD OF THE INVENTION

This invention is concerned with an electrode sensor kit for establishing electrical contact with skin comprising at least one contact element connectable to an analytical instrument. Furthermore, this invention relates to an electrode assembly for establishing electrical contact with skin comprising at least one contact element for forming a contact surface. Moreover, this invention describes the use of said electrode sensor kit or said electrode assembly for performing bio-signal measurements. The invention also is concerned .with the electro impedance tomography (EIT) imaging method comprising a step of applying contact elements to the skin surface for feeding electrical energy and/or measuring electrical signals. Furthermore, the invention also is concerned with a topical preparation provided with above kit and applied to the skin together and/ or at the same time with the electrical contact. Moreover, the invention is concerned with a method of establishing electrical contact with the skin of a living being and also with a method of determination of at least one electrical voltage or current value or an electrical voltage or current distribution on skin.

### BACKGROUND OF THE INVENTION

For intensive care doctors, pulmonologists, physiotherapists and high performance athletes electrical impedance tomography (EIT) is an imaging method that provides real-time information about regional lung ventilation and perfusion (flow or pulsatility of blood). In contrast to conventional methods, EIT does not require the patient to breathe through a sensor, does not apply ionizing x-rays and can be used for extended periods, say 24 hours or even longer. Therefore, EIT can be used continuously and is therefore suited for monitoring treatment and training effects in real time and along the time. EIT was first used to monitor respiratory function in 1983 and remains the only bedside method that allows continuous, noninvasive measurements of regional changes in lung volumes. Details about the EIT technique can be found in "Electrical impedance tomography" by Costa EL, Lima RG, Amato MB, Curr Opin. Crit. Care. 2009 Feb; 15 (1):18-24 or in Bodenstein M, David M, Markstaller K. Principles of electrical impedance tomography and its clinical application. Crit Care Med. 2009 Feb;37(2):713-24.

In EIT, an electrical current is applied to the skin of the thorax to establish an electrical field within the thorax. Typically, 8,16 or 32 electrodes are placed around the thorax und used to measure the electrical potentials resulting from the field. The measured voltages are used to estimate the distribution of electrical impedance within the thorax using algorithms specifically developed for ill-posed non-linear inverse problems. In order to overcome the ill-posed nature of impedance estimation, most EIT imaging algorithms make use of additional assumptions, known as regularizations, such as smoothness of the intra-thoracic impedance distribution, impedance contrasts or a-priory information on shapes and internal structures. These regularizations help the mathematical algorithm to decide between competing solutions, producing an image that is a reasonable estimation of the true impedance distribution within the thorax, at the expense of degraded spatial resolution or attenuation of maximum perturbations. Image creation software typically implements regularizations with different methods and such software is known in the art.

Finally, the calculated impedance-distribution is converted into an image that shows presence, absence or changes of gas and if desired also blood content. Plotted rapidly in sequence, like a movie, these images create a representation of gas and blood flowing in and out of each lung region and allow the doctor or athlete to evaluate lung function in real-time. Instead of plotting images, characteristic features can be extracted from the image and displayed as numbers or indices. Examples are: left vs. right ventilation or dorsal vs. ventral ventilation where these numbers represent for example a percentage of total ventilation.

The shape as well as the composition of the thoracic wall can contribute as much to the measured voltages at the chest wall surface as internal thoracic impedances. Consequently, the reconstruction of the absolute impedance distribution, albeit feasible, requires knowledge of the shape of the thorax as well as the impedance between the electrode and the skin.

Difference images, as first described by Barber and Brown, however can be generated without prior knowledge of the thoracic structure. They are generated from changes in impedance relative to a baseline or reference condition, assuming that both, the shape of the thorax as well as the contact impedances do not change significantly between these conditions. This relative or differential approach cancels out most errors related to incorrect assumptions about thoracic shapes, electrode position, body composition and contact impedances not only theoretically, but also in clinical practice with patients since this same error applies to both images in the same way. Most currently available EIT devices and most publications in the field use this relative approach. Thus, they display changes in impedance and not its absolute value. However, this perceived limitation is not a real problem if the dynamics of organ functions such as the beating heart and the breathing lungs are to be monitored.

However, even the use of relative EIT in clinical practice is not possible unless the contact impedance between electrodes and body skin become predictably stable over time. Any significant change of contact impedance will erroneously be perceived as changes within the organs of interest. Thus, even though the precise absolute value of the contact impedance at the site of each electrode does not need to be known, the condition that these values have to remain stable over time has yet to be fulfilled if meaningful EIT images are to be created by the image creation software.

Furthermore, it is highly desirable to not only achieve stable but also rather low contact impedances so as to make maximal use of the limited amount of current (10 mAmps) that is allowed to be injected into the living being. Only such currents achieve a maximal signal to noise ratio. When reconstructing images, traditional EIT algorithms assume that the electrodes (usually 8,16 or 32) are located at discrete physical locations around the chest, most often in an equidistantly spaced fashion. They do not take crosstalk between such electrodes into account. Crosstalk and changes in crosstalk can be interpreted by the image creation software as a signal stemming from internal organs and functions of these organs. Thus, crosstalk between electrodes should remain low and constant.

Yet another aspect to be considered when designing any device or structure to be placed in direct contact with the skin of a living being is its physical impact on such skin, which may lead not only to a physical irritation or even breakdown of the skin, but also of the underlying tissues such as muscles, tendons or bones (decubitus). The key contributing factors in the pathogenesis of such breakdown are: 1) acute critical or chronic illness of patient (intrinsic factors), 2) the local absolute pressure (usually > 30 mmHg) compressing the small blood vessels and capillaries (this is usually the highest in areas where bones are located close to the body surface) and/or the pressure relative to the one perfusing the tissue, 3) the time such pressures are applied, and 4) the time-pressure-product (even very high pressures can be tolerated for short periods of time). Furthermore, 5) elevated moisture and 6) elevated temperature levels with increased metabolic demand make the tissue susceptible to damage. In addition, 7) shear stress (forces tangential to the tissue surface) exert their negative effects mainly in the capillary region where they lead to a kinking of such vessels preventing oxygen and nutrient rich blood from flowing to the site of utilization (ischemia).

In the context of the above, it becomes obvious that any physical structure such as an electrode applied to the fragile skin of living subjects preferably should accommodate the individual needs of such subjects, especially those of their skin. Such needs preclude the use of any non-permeable occlusive physical structure such as a belt made of silicone, rubber or plastic carrying electrodes made of electrically conductive silicone, metal or other electrically conductive material, or any gel or sticky tape that prevents the skin from "breathing naturally" and from exchanging moisture (essentially no trans epidermal water loss = TEWL) and heat for extended periods of time such as days or weeks.

Traditional EIT systems use adhesive gel electrodes, which when in contact with the warm and moist skin of a patient may change their electrical characteristics quite drastically. Gels are finely dispersed systems consisting of at least one sponge-like solid and one liquid phase (typically 90 wt-% water or more). The pores within the solid phase are filled with the liquid, which over time will evaporate leading to a loss of water if such losses are not replenished from within the skin. Thus, gel electrodes can promote the loss of water within the already rather dry cover layers of the skin, which in turn will increase their resistance to electrical currents. In an attempt to make up for these increased resistances gel electrodes typically contain silver-silver chloride as electrically conducting ions. These ions in turn can create an osmotic pressure, which again causes a net flux of water towards them leading to further losses of water from within the skin. Furthermore, the typical gel electrode is designed such that in the very center of the gel contact is established to an electrical wire leading to the typical push button to which cables are attached. This minimal electrical contact surface between the electrically perfectly conducting metallic part of the electrode and the poorly conductive gel leads to a very limited electrically effective electrode area. Thus, while the structural dimension of the gel pad might appear large, its electrically active surface is usually not, which inevitably leads to a high electrical resistance. After the electrode, which is usually stored at room temperature, has been applied to the warm skin its resistance increases significantly even further with every degree.

Alternatively, instead of gel electrodes belt-like fixtures are currently in use in particular for EIT applications. These fully occlusive electrode arrangements consist of a wide silicone strip, which contains within its inner wall 8,16 or even 32 electrode areas made of electrically conductive materials such as carbon impregnated silicone. In order to achieve adequate physical contact between the individual electrodes and the subject's skin the belt must be tightly wrapped and fixed around the body. Due to the lack of moisture or free fluids on such belts the initial electrical contact with the patient's skin is not optimal, but will improve over time as sweat and moisture accumulate. To obtain adequate contact conditions right from the start, contact gels can be applied. While from an electrical point of view such a fully occlusive design might be advantageous, it cannot be used for extended periods of time as the skin will inevitably swell and be destroyed by pressure, moisture, heat and if used also by the constituents of the gel. For these reasons, the use of such belts cannot be recommended for uses longer than 4 hours. Thus, such designs do not fulfill the essential requirements of a skin-friendly EIT electrode arrangement. The same limitations apply to dry metal electrodes of any type.

From a theoretical as well as from a practical point of view it would thus be highly desirable for any EIT application to obtain direct access to the inner fluidic compartment of the body by overcoming the electrical barrier imposed by the natural outer skin, the "epidermis", in particular is outermost layer, its "stratum corneum" consisting of dry and dead former epithelial cells. Two obvious ways to overcome this natural barrier - which biology designed to prevent the body from excessive water losses and to protect it against mechanical stress and strain - imaginable are for example are 1) a physical removal of at least the outermost skin layer by systematic abrasions in places where electrodes shall be placed or 2) a penetration of the skin by sticking through it needle-like electrical conductors. For obvious reasons such destructive methods cannot even be conceived for clinical use in sick patients.

Dry textile electrodes have been used to establish electrical contact between the wearer's skin and electronic devices, such as heart rate monitors for runners, however, these electrodes work only after the individual has begun to sweat significantly in the areas where such electrodes are applied. As soon as this interface dries again, the electrical contact may be lost. While these knitted or woven textile solutions are convenient and pleasant to wear and permit transpiration, their electrically active surface areas are usually very small compared to the overall physical electrode surface area due to the poorly conductive synthetic yarns used and the limited amounts of contact points with the skin resulting from the large dimensions of the yarns and the typical textile production processes used. This is why such electrodes either require high contact pressure, or need to be rather large and thus do not lend themselves to applications in fields such as EIT where 8,16 or 32 electrodes need to be placed within the limited perimeter of i.e. a chest wall.

For the reasons described above, there is a clear need for methods and means to achieve a more reliable and stable electrical contact between the skin of a living being and the electrodes of EIT systems. At the same time such means and methods need to take into account the fragile nature of the skin. Moreover, any potential solution must be producible in large quantities and at low costs, especially when designed as typical single patient use disposable items.

It is known that "contact" impedances between the electrode and skin render the accurate measurement of the underlying tissue impedance difficult (see E.T. McAdams et al., Factors affecting electrode-gel-skin interface impedance in electrical impedance tomography", Medical & Biological Engineering & Computing (Nov. 1996), pages 397 - 408). In order to ensure an optimal electrical contact electrode gels may be used. Such gels can be divided into so-called "wet" gels and hydrogels. Standard gels are usually composed of water, a thickening agent, a bactericide, fungicide, an ionic salt and a surfactant. Thereby the ionic salt serves to ensure the electrical conductivity of the gel. However, as the human tissue cannot tolerate long-term exposure to salt concentrations which depart significantly from physiological levels, so-called aggressive gels with NaCl concentrations > 5% should not be used.

Hydrogels are "solid" gels which incorporate natural (e.g. karaya gum) or synthetic (e.g. polyvinyl pyrrolidone) hydrocolloids. According to the authors hydrogels are hydrophilic, are poor at hydrating the skin and may even absorb surface moisture. They tend to be more resistive than standard "wet" electrodes. Typical resistivities for 'wet' electrodes tend to be in the range between 5 - 500 Ωcm⁻¹ compared to 800 - 8000 Ωcm⁻¹ for hydrogels.

In order to reduce the barrier properties of the skin it has been proposed to use so-called 'penetration enhancers'. Although the mechanisms by which the penetration enhancers function are not well understood, it is assumed that in some cases they may alter the hydration of the stratum corneum or alter the packing structure of the ordered lipids in the intercellular channels (Knepp et al. 1987, Transdermal drug delivery: problems and possibilities in CRC Crit. Rev. Therapeutic Drug Carrier Syst., 4 (I), pp. 13-37).

A class of penetration enhancers often used in electrolytic gels is surfactants. Such surface active agents are adsorbed at water-oil interfaces as a result of their hydrophilic (or polar) groups and lipophilic (or non-polar) groups. By orientation at a water-oil interface, the molecules of the surfactants facilitate the transition between polar and non-polar phases. The use of esters of saturated and unsaturated fatty acids and of natural oils has also been reported. A 0.2% solution of lauryl sulfate could reduce theelectrical resistance of the stratum corneum by 95%. However, it is also reported by .T. McAdams et al. that, although the use of a given penetration enhancer may increase the skin's permeability to certain drugs, it does not always follow that the skin's electrical impedance will be decreased.
US 2005/0136077 relates to a composition providing electrically conductive adhesive hydrogels suitable for use as an electrical interface for disposable medical devices. Said hydrogels provide for reduced skin irritation, hydrate a subject's skin and readily wet around a subject's skin surface hair. The composition of the hydrogel comprises a monomer, a first initiator at a first concentration, a second initiator at a second concentration and a cross-linking agent. The hydrogel can optionally comprise a conductivity enhancer in the form of inorganic salts like potassium choride, sodium chloride etc. or salts of weak organic acids like sodium citrate and magnesium acetate. The conductivity enhancer will be present in an amount between 0 and 15% by weight of the hydrogel precursor.
US Patent No. 3,567,657 relates to an electrically conductive material selected from the group consisting of benzoic, salicyclic, tartaric, citric, lactic and malic acids and the sodium and potassium salts thereof. One exemplary composition of an emulsion type comprises sodium tartrate (3.5 %), fatty acid groups (6.5 %), natural or synthetic oils (4.0 %), triethanolamine (3.0 %), glycerine (4.0 %) and water (79%).
WO 2010/078441 discloses an electrode assembly for neuro-cranial stimulation. It includes an electrode, a conductive gel and an adapter for positioning the electrode and for receiving and retaining the conductive gel. The gel preferably contains 1) a polymer, which functions include support properties, 2) surfactants or surface acting agents, functioning to act on the skin to increasing permeability and/or change of skin resistivity, 3) humectants, functioning to maintain gel hydration, 4) salts, functioning to increase electrical conductivity, 5) water, and 6) preservatives or other chemicals. The preferred surfactants have oil solubilizing properties and can be ionic and non-ionic surfactants like sodium hexametaphosphate. Suitable salts are ionizable salts, salts of acids or bases or buffer solutions. Examples of inorganic salts include potassium chloride, sodium sulfate and organic acids or salts such as citric acid potassium citrate, or potassium acetate. Among the additive agents appear natural oils like coconut or castor oil, Aloe Vera, synthetic beeswax etc. These agents act to protect or restore the skin.

### OBJECTIVE OF THE INVENTION

The objective of the present invention is the provision of a reliable and stable electrical contact between electronic measuring equipment, in particular the electronic part of an electrical impedance tomography device, and the skin of a living being, e.g. a test person or patient. Furthermore, the objective of the present invention is a new and improved electrical patient interface for electrical impedance tomography which minimizes and stabilizes the electrical contact impedance between the skin and the electrodes of an EIT device. A further objective is the provision of reliable electrical contacts to the skin while at the same time avoiding skin break down and/or cross-talk between electrodes. A further objective is the provision of numerous electrical contacts to the skin, preferably for use in the EIT technique. A further objective is to obtain a skin contacting device that is producible at low cost, particularly for single-patient use products.

### SUMMARY OF THE INVENTION

Above objective is solved with an electrode sensor kit, an electrode assembly, the use of the electrode sensor kit or the electrode assembly, an electro impedance tomography imaging method, a topical preparation used therewith, a method of establishing electrical contact with the skin of a living being, and a method of determination of electrical voltage or current values on skin and/or electrical voltage or current distribution on skin, as disclosed in the following.

The invention is defined in the appended claims.

The inventive electrode sensor kit for establishing electrical contact with skin comprises the following components:
- at least one contact element (such as e.g. an electrode or a senor plate) which is connectable to an analytical instrument,
- a preparation comprising a mixture of water and at least one lipid for enhancing electrical contact properties between said contact element and the skin, and
- said mixture forming an emulsion (for example an oil-in-water or a water-in-oil emulsion), wherein said preparation has a conductivity of less than 1mS/cm.
The connectable contact element may for example comprise an electrically conductive plug or socket position, wires, and/or cables for establishing communication with an analytical instrument.

This kit provides the components for an electrode sensor assembly as defined in claim 13. With this inventive kit the electrical properties of the skin-electrode impedance are optimized by interposing an active interface in-between the surfaces of a contact electrode and the skin. Hereby the active interface consists of said preparation, i.e. a topical preparation for application to the skin. Thus, the impedance of the wearer's skin is optimized by applying said preparation, which may be made available e.g. in the form of a liquid, a cream, a gel, or an ointment.

In a preferred embodiment the inventive electrode sensor kit comprises a plurality of contact elements, in particular for forming an assembly of a plurality of electrode sensor plates.

Advantageously, the electrode sensor kit is characterized in that said preparation is essentially non-conductive. In particular the preparation is free from electrolytes, such as salts, which would lead to considerable electrolytic conductivity of the preparation.

Thus, the electrode sensor kit comprises a preparation which is essentially salt-free. Essentially salt free means hereby that the salt content is such that the electrical conductivity of the preparation is below a predetermined value. The electrode sensor kit is characterized in that conductivity of the preparation is less than 1 mS/cm (*milli-siemens per centimetre*).

Advantageously the electrode sensor kit is characterised in that said preparation comprises at least an active ingredient selected from the group consisting of hygroscopic substances, hydrophilic substances, saccharides or polysaccharide, polyacry-lates, panthenol or D-panthenol, allantoin, aloe vera, glycosaminoglycans, anionic nonsulfated glycosaminoglycans, algae or alginic acid, amino acids or proteins, and hyaluronic acid or salt; whereby hyaluronic acid is preferred.

Preferably, the preparation comprises at least an alcohol, preferably an alcohol selected from the group consisting of mono-, di-, tri-, and polyhydroxy alcohols, glycerol, sorbitol, propylene glycol, and combinations thereof.

Advantageously said at least one lipid is selected from the group consisting of oils, preferably vegetable oils; phospholipids, preferably diacylphospholipids, phosphatidylcholine, phosphatidylethanolamine, phosphatidylglycerol, phosphatidylserine, phosphatidylinositol, and their monoacyl derivatives; cholesterol; natural lecithins, preferably natural lecithins from egg, milk, soy, sunflower, and/or oat; enzyme hydrolysed lecithins, preferably enzyme hydrolysed soy lecithins; mixtures of monoacylphospholipids and diacylphospholipids, said mixtures preferably containing 10 to 90 percent by weight of monoacylphospholipids; and combinations thereof.

Preferably, said at least one lipid and/or active ingredient is present in the form of liposomes or nano-particles.

Advantageously, the shell of the nano-particles include lipids as membrane components and optionally essential fatty acids, such as e.g. linoleic acid.

The preparation according to present invention may be present in the form of a fluid, a gel, or a cream.

Emulsifying agent may be used with caution and at the lowest possible concentration but should whenever possible be avoided as they may destroy the integrity of the skin structure and lead to a washout of lipophilic components.

For the purpose of present application the gel can be considered as a substance that contains a continuous solid phase forming a three-dimensional skeleton and enclosing a continuous liquid phase. This definition is based on C.J. Brinker and G. W. Scherer in "So-Gel Science", p. 8, 1990. The solid skeleton may be formed of e.g. polymeric and/or particulate material whereby the bonds within the gel may be established by covalent bonds (e.g. forming polymeric gels), by Van-der-Waals forces (e.g. particulate gels) or a combination thereof. By weight of their composition, gels are usually mostly liquid, yet they behave like solids due to the three-dimensional network within the liquid. The term "continuous" in above context means that one could travel through one phase from one side of a sample to the other without having to enter the other phase. For forming a gel e.g. the water and lipid of above preparation are combined with a skeleton generating substance.

For the purpose of present application a cream can be considered as a semi-solid emulsion which is a mixture of oil and water. Depending on the composition creams may be divided into two types: oil-in-water (O/W) creams which are composed of small droplets of oil dispersed in a continuous aqueous phase, and water-in-oil (W/O) creams which are composed of small droplets of water dispersed in a continuous oily phase. Oil-in-water creams are less greasy and more easily washed off using water. Water-in-oil creams are more moisturizing as they provide an oily barrier which reduces water loss from the outmost layer of the skin.

The mixture of water and at least one lipid forms an emulsion, in particular an oil-in-water emulsion or a water-in-oil emulsion.

Creams and gels may be considered pharmaceutical products (i.e. ointments).

The use of the Finger Tip Unit (FTU) concept may be helpful in guiding how much topical cream is required to cover different areas, e.g. the area per electrode contact element. In medicine, a finger tip unit (FTU) is defined as the amount of ointment, cream or other semi-solid dosage form expressed from a tube with a 5mm diameter nozzle, applied from the distal skin-crease to the tip of the index finger of the individual adult patient. One FTU is enough to treat an area of skin twice the size of the flat of the adult patient's hand with the fingers together, i.e. a "handprint". One handprint is 0.8% (i.e. approximately 1%) of the total body surface area, and one FTU covers approximately two handprints. As two FTUs are approximately equivalent to 1g of topical application, the "Rule of Hand" states that "4 hand areas = 2 FTU = 1g" (modified from: Finlay AY, Edwards PH, Harding KG. "Fingertip unit" in dermatology. Lancet 1989; II, 155 and Long CC, Finlay AY, Averill RW. The rule of hand: 4 hand areas = 2FTU = 1g. Arch Dermatol 1992; 128:1130-1131.

One to three finger tip units (equal to about 0.5 to 1.5 grams) of cream or gel usually is required to cover effectively the thoracic zone on which the electrodes are to be applied. In an adult, this covers an area in the range of about 300 to 900 square centimeters of skin.

Conveniently, the amount of the at least one lipid in the preparation is in the range of 10 to 90 weight percent, preferably in the range of 30 to 85 weight percent, and more preferably in the range of 50 to 80 percent. Conveniently, the amount of water in the preparation is in the range of 5 to 95 weight percent, preferably in the range of 10 to 60 weight percent, and more preferably in the range 15 to 40 weight percent.

However with regard to the conductivity values achievable, most advantageous preparations are oil-in-water preparations. Such oil-in-water preparations comprise e.g. an amount of the at least one lipid in the range of 5 to less than 50 weight percent, preferably in the range of 10 to 45 weight percent, and more preferably in the range of 15 to 40 percent. Hereby the amount of water is preferably in the range of 50 to 90 weight percent, more preferably in the range of 50 to 85 weight percent, even more preferably in the range of 50 to 80 weight percent. Further preferred respective lower limit of the amount of water is 55 weight percent, and more preferably 60 weight percent.

Advantageously said at least one contact element comprises a material selected from the group consisting of metals, conductive polymers, textiles and conductive textiles, or a combination thereof.

An electrode assembly usable for electrical impedance tomography comprises the components of the kit wherein (a) said at least one contact element forms an electrode or sensor plate, and (b) said at least one contact element comprises a layer of said preparation.

Thus, an electrode assembly for establishing electrical contact with skin usable for electrical impedance tomography comprises
- at least one contact element connectable to an analytical instrument, said at least one contact element forming an electrode or sensor plate, and
- a preparation comprising a mixture of water and at least one lipid for enhancing electrical contact properties between said contact element and the skin, said mixture forming an emulsion, in particular a water-in-oil or an oil-in-water emulsion, wherein said at least one contact element comprises a layer of said preparation.

Preferably said electrode assembly is further characterized in that
- the preparation is a fluid, a gel, or a cream,
- the preparation is incorporated into or applied onto a conductive fabric,
- the preparation forms an interface layer to the skin of a living being.

The preparation's electrical conductivity is less than 1 mS/cm.

Advantageously the electrode assembly is characterised in that the at least one contact element comprises a surface for contacting the skin, said surface being coated or impregnated with the preparation for enhancing electrical contact properties.

The electrode assembly further may be characterized in that a plurality of contact elements are arranged on or integrated in a belt-like structure. Typically, the contact elements are lined up in sequence forming an array.

An electrode assembly for establishing electrical contact with skin, advantageously, is characterized by at least one contact element and a preparation, said preparation comprising a mixture of water and at least one lipid, and further characterised in that the at least one contact element comprises a surface for contacting the skin, said surface being coated or impregnated with the preparation for enhancing electrical contact properties between said contact element and the skin. Preferably the electrode assembly comprises at least the contact element and the preparation of above described electrode sensor kit.

Preferably the electrode assembly is characterized in that said surface for contacting the skin is structured. The structured surface is uneven, pocketed and/or porous.

A particularly advantageous embodiment is the electrode assembly for electrical impedance tomography comprising
- an electrode or sensor plate;
- a layer of essentially electrically non-conductive preparation which forms the interface to the skin of a living being;
- wherein said essentially electrically non-conductive preparation is a fluid, a gel, or a cream;
- wherein said layer of essentially electrically non-conductive preparation is incorporated into or applied onto a conductive fabric;
- wherein the preparation's electrical conductivity is less than 1 mS/cm (*milli-siemens per centimetre*) and
- wherein said layer of essentially electrically non-conductive preparation is composed of at least water and a lipid forming an oil-in-water or a water-in-oil emulsion.
Said fabric serves for contacting the skin of a living being.

The use of the electrode sensor kit or the electrode assembly for performing bio-signal measurements is characterised in that said preparation and said at least one contact element are applied to the skin of a test person, so that the preparation is interposed between skin and the at least one contact element during EIT measurement.

Advantageously several electrode elements are used for performing the measurement, in particular when performing EIT measurements. Preferably the several electrode elements are lined up in succession, such that each neighboring electrode is spaced apart in a distance of 0.5 cm to 10 cm, preferably in a distance of 1 cm to 5 cm, from its two neighbors. Such choice of electrode distance assures correct measurement essentially excluding cross-talk and at the same time allowing for a sufficient data point density, e.g. when taking measurements with electrodes which are arranged around a patient's chest.

Advantageously the electrical conductive properties between skin and electrode sensor pad is adjusted by inducing controlled sweating, preferably by appropriate parasympathomimetic drugs belonging to any one of the following three general groups:
1. cholinesters (esters of choline) such as e.g. acetylcholine, carbachol (carbamylcholine), bethanechol (carbamylmethylcholine), or metacholin;
2. parasympathomimetic alkaloids such as e.g. pilocarpine; and/or
3. reversible cholinesterase inhibitors (also called "anticholinesterase") such as e.g. physostigmine (eserine), neostigmine, pyridostigmine, distigmine, or demecarinum.

Preferably for use of the electrode sensor kit or the electrode assembly, a sweat inducing topical medication is applied to the skin, e.g. either with the preparation or separately thereof, with said sweat inducing topical medication comprising preferably the non-selective muscarinic receptor agonist pilocarpine while alternatively or in addition the cholinesterase inhibitors physostigmine and neostigmine can also be used.

The latter topical medications can be applied alone, together with adrenaline, which further enhances sweat gland activity, or in variable combinations amongst these medications such as the typical combination of pilocarpine and physostigmine.

Advantageously, the preparation contains a sweat enhancer, e.g. the topically applied sweat inducing medication pilocarpine which is delivered down to the sweat glands within the skin by way of iontophoresis. Iontophoresis is a technique using an electric charge to deliver a medicine or other chemical through the skin. The electrical charge is applied locally and usually relatively low in order not to damage the skin but sufficiently high in order to transport the medicine or chemical.

Preferably, the bio-signal measurements are selected from the group consisting of EIT-measurement, heart-rate-measurement, and ECG-measurement.

The method of establishing electrical contact with the skin of a living being comprising application of contact elements to the skin surface for feeding electrical energy and/or measuring electrical signals is characterized in that
- a preparation is applied to the skin at locations where the contacting elements are to be applied, and
- the preparation comprises a mixture of water and at least one lipid for enhancing electrical contact properties between said contact elements and the skin.

Advantageously said method of establishing electrical contact with the skin uses above-described electrode sensor kit, in particular above-described electrode assembly.

The EIT imaging method comprising application of contact elements to the skin surface for feeding electrical energy (i.e. in the form of electrical currents) and/or measuring electrical signals (i.e. in the form of electrical surface potentials) is characterized in that
- a preparation is applied to the skin at locations where the contacting elements are to be applied, and
- the preparation comprises a mixture of water and at least one lipid for enhancing electrical contact properties between said contact elements and the skin, and
- optionally the preparation comprises a sweat inducing medication for enhancing electrical contact properties between said contact elements and the skin.

Advantageously an EIT imaging method employs above-described electrode sensor kit, in particular above-described electrode assembly.

The inventive topical preparation serves for enhancing and stabilizing electrical contact properties of the skin. This topical preparation comprises a mixture of water and at least a lipid, wherein said mixture forms an oil-in-water or water-in-oil emulsion.

The topical preparation may comprise at least an additive selected from the group of functional additives consisting of hyaluronic acid or salt, hygroscopic substances, hydrophilic substances, saccharides or polysaccharide, polyacrylates, panthenol or D-panthenol, allantoin, aloe vera, glycosaminoglycans, and anionic nonsulfated glycosaminoglycans, algae or alginic acid, amino acids or proteins and hyaluronic acid or salt; whereby hyaluronic acid is preferred.

Advantageously the topical preparation is essentially non-conductive. This means that the conductivity of the topical preparation is less than 1 mS/cm (*milli-siemens per centimetre*).

It is preferred that the topical preparation comprises at least an alcohol, preferably an alcohol selected from the group consisting of mono-, di-, tri-, and polyhydroxy alcohols, glycerol, sorbitol, propylene glycol, and combinations thereof.

Optionally additives may be comprised in the topical preparation. Such additives comprise skin compatible surfactants (surface active agents), humectants, odorants, and/or colorants etc.

However, it is preferred that the topical preparation is essentially free of lauryl sulphates or other detergents or surfactants which potentially damage a patient's skin. This is especially relevant when measurements are performed repetitively and/or over a long period, such as e.g. several hours or several days. It is especially preferred that the topical preparation is essentially free of ionic detergents or ionic surfactants, in order to keep the conductivity of the topical preparation below the limits described above, i.e. essentially in a non-conductive range; whereas non-ionic detergents and/or non-ionic surfactants may optionally be contained in the topical preparation.

Preferably the topical preparation is characterised in that the at least one lipid is selected from the group consisting of oils, preferably vegetable oils; phospholipids, preferably diacylphospholipids, phosphatidylcholine, phosphatidylethanolamine, phosphatidylglycerol, phosphatidylserine, phosphatidylinositol, and their monoacyl derivatives; cholesterol; natural lecithins, preferably natural lecithins from egg, milk, soy, sunflower, and/or oat; enzyme hydrolysed lecithins, preferably enzyme hydrolysed soy lecithins; mixtures of monoacylphospholipids and diacylphospholipids, said mixtures preferably containing 10 to 90 percent by weight of monoacylphospholipids; and combinations thereof.

The topical preparation may be in the form of a fluid, a gel or a cream.

The topical preparation may be used as medicament to enhance electrical contact properties of the skin, in particular for enhancing the electrical conductivity of the skin.

The topical preparation may be used in an electrical diagnostic method, which electrical diagnostic method includes a step of feeding electrical energy to the skin and/or measuring electrical signals on the skin.

Moreover, the topical preparation may be used in an electrical diagnostic method, which electrical diagnostic method includes a step carried out by a measurement method selected from the group consisting of electro impedance tomography (EIT), heart-rate-determination, and electro cardiograph (ECG) measurements.

A method of determination of at least one electrical voltage or current value or an electrical voltage or current distribution on skin is characterized in that a topical preparation comprising an oil-in-water or water-in-oil emulsion formed from a mixture of water and at least a lipid is applied to the skin at predetermined locations prior to application of electrical current or voltage and/or measurement of electrical values at said predetermined locations.

Said method, preferably, is characterised in that said topical preparation and at least one contact element are applied to the skin of a living being, so that the topical preparation is interposed between skin and the at least one contact element during the measurement.

Advantageously said method may be characterized in that said values are determined by at least one of a measurement selected from the group consisting of electro impedance tomography (EIT) measurements, heart-rate-measurements, and electro cardiograph (ECG) measurements.

### DESCRIPTION OF THE INVENTION

The electrical properties of a skin-electrode contact area are optimized by a new and inventive patient interface comprising a mediating preparation applied to the skin surface of a patient or test person. The preparation advantageously comprises a fluid, cream, or gel. Advantageously, the preparation may comprise further a medication such as e.g. a sweat inducing medication or a wound healing medication. Thus, the mediating preparation may for example comprise an ointment. A contact surface of an electronic circuit, i.e. an electrode surface, which establishes contact between the skin and the electronics (such as e.g. an EIT instrument) is set in physical contact with the above prepared skin surface.

The preparation preferably is composed in such a way that it is electrically minimally conductive or even non-conductive (i.e. the resistivity of the preparation medium is more than 1000 Ohm*cm). The preparation is used as solvent for those electrolytes, i.e. salts, which are provided by the natural skin itself. Especially for areas of application, in which arrays of electrodes are use, such as EIT, the low conductivity is particularly preferable. This is because a high intrinsic electrical conductivity of the preparation, such as a conductance higher than the one of skin, i.e. higher than 10 mS/cm could lead to enhanced crosstalk between adjacent electrodes and consequently to erroneous EIT data.

The inventive preparation contains both water and lipids. Thus, it is a kind of emulsion. Without being bound to any theory, it is believed that, while the water within such preparations is used to establish an immediate and reliable electrical contact between the electrode and the skin by dissolving the electrolytes residing on or within the patient's skin, the lipids are used to establish a shielding layer (the shielding layer herein also referred to as occlusion, occlusive layer or occlusive shielding), which reduces or prevents body water from leaving through the skin, thus keeping trans epidermal water loss (TEWL) low. Even more, the occlusive layer does not only prevent water loss from within the body, but more importantly, it moves the transitional zone between wet and dry skin compartments outwards, ideally all the way up to the skin surface.

Thus, instead of abrading the skin or penetrating it with conductive pathways, it is believed that the lipid layer, i.e. the occlusion, actively changes the electrical properties of the outer skin in such a way that it becomes electrically similar or even equal to the milieu of the inner body - the actual target of the EIT measurements - thereby removing the natural barrier created by the dry outer skin layers. It is assumed that, as the water provided by the emulsion evaporates over time, its lipid component covers the treated skin surface. The occlusion seems to allow very little water from within the body to be lost at the skin surface and to promote water diffusion into the upper skin layers where it not only changes local humidity levels towards full saturation, but also dissolves the vast amount and high concentration of free ions residing especially within the uppermost zone where active ion transports cease as the cells die. Thus, the occluded skin not only appears to attract water from within the body but also becomes more conductive presumably as residual ions are dissolved.

In a preferred embodiment the preparation is an emulsion that contains when being applied a water content of maximally 90 wt-% and a lipid content of maximally 90 wt-%.. While excessive fractions of non-conductive lipids lead to an electrical insulation and thus poor contact impedance, an emulsion containing too much water will be unstable over time since its water will be lost by evaporation, which again increases contact impedance. Thus, the right mixture will be in the order of 10 wt% to 90 wt-% lipid and 10 wt-% to 90 wt-% water.

In one embodiment the electrical contact between the skin and an electrode is improved by actively inducing local sweating by means of appropriate medications such as parasympathomimetic or acetylcholine-like drugs (such as e.g. the non-selective muscarinic receptor agonist pilocarpine) applied onto the skin. Pilocarpine is a non-selective muscarinic receptor agonist, which acts therapeutically at the muscarinic acetylcholine receptors including the ones of the sweat glands. However, it is well known that the highly polarized molecules do not easily diffuse through the skin down to its site of action i.e. at the root of the sweat glands if applied topically. Thus, preferably the drug, such as pilocarpine, is actively delivered to the desired location by applying a direct current (DC) that drags this drug along; this methodology being called "iontophoresis". Once attached to the receptors at the glad, the drug induces sweat production until it is neutralized by acetylcholine esterases, other enzymes or non-enzymatic mechanisms such as Hofmann-Elimination. The typical half life for its sweat-inducing action is around one hour.

It was found that for short experiments of maximally up to one hour the application of pilocarpine is useful in order to reach a skin conditions with low and stable skin resistance and therefore reproducible impedance values. In experiments lasting longer than one hour large impedance variations were found, when relying on pilocarpine alone for the production of sweat, i.e. humidity. From this it can be concluded that the addition of pilocarpine or any similarly acting parasympathomimetic alkaloid is most advantageous for EIT-measurements of short duration, i.e. up to one hour. However, the use of pilocarpine, in particular in combination with the inventive preparation, may also be advantageous for long term measurements of over one hour. Here the pilocarpine assists in reaching an early stabilization of the skin condition, i.e. sufficient conductivity of the skin, so that immediately (i.e. less than 5 minutes) after application of the preparation including pilocarpine reproducible measurement data may be received while later on the lipid components exert their occlusive stabilizing effects.

In a further embodiment, pilocarpine or similar sweat inducing medications are applied repeatedly preferably by repeated periods of iontophoresis so as to achieve a constant conductivity level at the skin surface.

In a further embodiment the conductivity between the skin and an electrode is further improved by actively inducing local sweating under and in the close vicinity of an electrode. For this purpose a sweat inducing drug may be applied to the outer skin in the area where the electrode shall be placed. Advantageously in addition, through an electrode pair electrical DC current is applied to the treated skin in order to force transport the drug to its site of action. Hereby, possibly the drug penetrates deeper into the skin than by simple spreading of the drug or a preparation containing the drug onto the skin surface. Preferably, the very same electrode, i.e. the electrode of which the electrical contact to the skin shall be improved, together with another electrode, i.e. a counter-electrode, attached to any location of the body can be used to apply the electrical DC current across the treated skin surface area. In EIT arrays 8,16 or even 32 electrodes are typically used and therefore, sequential and suitable combinations of these EIT electrodes can be used for the above DC iontophoresis purposes prior to or during their use as EIT electrodes whereby their skin-electrode electrical contact properties are optimized.

While the electrical barrier of the natural skin is overcome by DC iontophoresis without physically destroying its fragile structure, such interventions are not sufficient to achieve an optimal and stable overall electrical contact between skin and electrode. Further measures are required.

The electrical properties of a skin-electrode contact area may be further optimized by an interface layer which increases the electrically active surface area of such contact, when bridging interposed between skin and electrode.

Thus, another embodiment is concerned with the deliberate increase of the electrically active surface area between the skin and the electrode. This challenge is solved by interposing a layer of a hygroscopic and/or hydrophilic material with a large surface area between the skin and the electrodes in order to retain the electrolyte-containing aqueous body fluid, which is formed with time after application of the preparation to the skin. Different materials can be used such as foams, solid gels, woven, non-woven fabrics or any porous material sufficiently inert to water and body fluids. Depending on the thickness of this interposed layer, this material may be electrically conductive or non-conductive. While very thin layers of non-conducive materials (i.e. smaller than 1 mm or more advantageously smaller than 0.1 or even smaller than 0.05 mm) will become fully soaked with above body fluid and thus establish a perfect electrical contact with the electrode surface, thicker layers need to be made of electrically conductive materials as only their skin-contacting surface might become wetted and the remainder of the material will have to establish the electrical pathway towards the actual electrode.

As the electrical properties of such a pathway depend on the layer material used, it is advantageous to optimize also its electrical coupling to the actual electrode. Such contact is optimized if the main direction of the currents flowing through these electrical pathways runs orthogonal to the surface area in the Z-direction. As the electrode surface area is defined by its x and y dimensions, the currents flowing along the X and Y vectors should be minimal if the electrical conductivity of the interface layer is significantly lower than that of copper. Thus, the electrical properties of the contact impedance between the electrode and an interface layer made of poorly conductive material are optimized towards the lowest possible values by matching as best as reasonably possible the dimensions and the actual mutual fit of the respective areas of contact. This way the electrically active surface of the electrode made of highly conductive material such as copper, gold, palladium, platinum or other materials of that kind spreads the current over its entire surface area while the flat and thin interface layer directs this current through its shortest dimension directly into the wearer's skin. This way the overall performance of the electrode-skin interface is optimized.

Furthermore, the above interface layer is designed such that it prevents excessive loss of water from the skin surface by means of evaporation thereby ensuring long-term stability of the electrical contact properties. The interface layer thus acts as a "second skin" or more precisely in a similar manner as the outer dry skin layer. Using such an approach, the inner milieu of the body moves outward into the manmade structures that now become easily electrically accessible.

### BRIEF DESCRIPTION OF THE DRAWINGS

An embodiment of the present invention will now be described by way of example with reference to the accompanying drawings showing schematically in
- Figure 1:: a cross sectional view of an electrode sensor kit;
- Figure 2:: a frontal view of a representative section of electrode assembly;
- Figure 3: experimental setup.

### DETAILED DESCRIPTION OF THE DRAWINGS

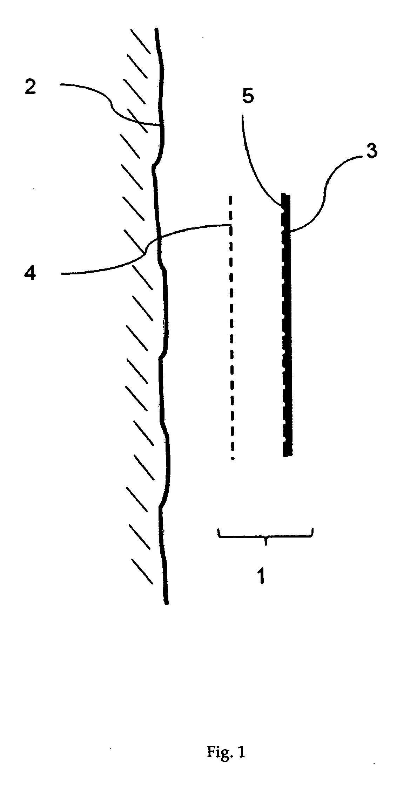

In Figure 1 an exploded view of a cross sectional view of an electrode sensor assembly 1 according to the invention is schematically shown in relation to a living being's skin 2. The electrode sensor assembly 1 comprises a contact element 3. Said contact element 3 comprises an electrically conductive material. On the side of the contact element 3 facing the skin 2 (i.e. surface 5 of contact element 3), the contact element 3 is in contact with preparation 4. Contact element 3 and preparation 4 form said sensor assembly 1. The contact element 3 may comprise a porous structure and/or layer on at least one surface 5. Exemplarily said porous structure comprises a fabric, preferably a conductive fabric. The preparation may penetrate into said pores, e.g. into said fabric. Furthermore the contact element 3 is connectable to an analytical instrument (not shown here).

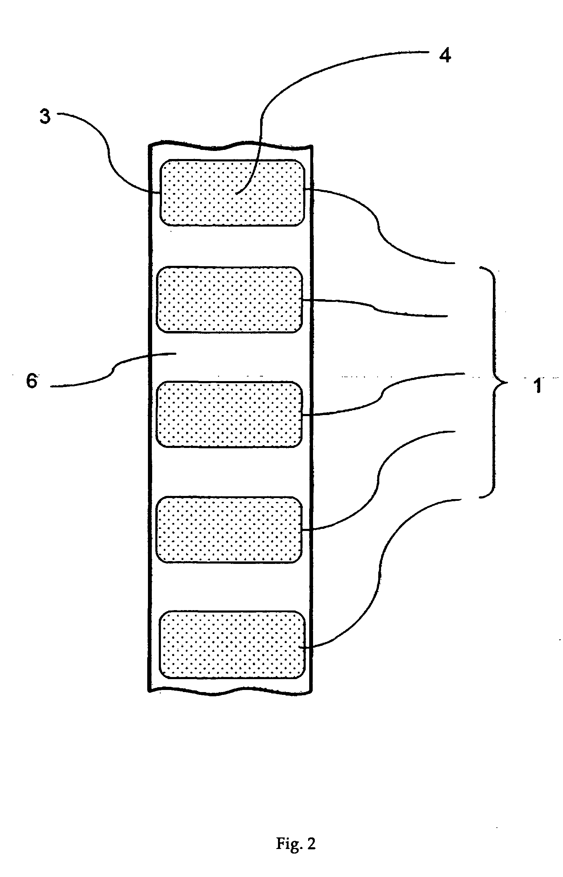

In Figure 2 a frontal view of an electrode assembly 1 for electrical impedance tomography is depicted. The electrode assembly 1 comprises several plate-like contact elements 3 which are attached to or integrated in a belt-like strap 6, such as for example a strip, in particular a strip of cloth, a belt, or a band. The contact elements 3 are arranged in mutual spaced apart manner. Advantageously said arrangement extends in longitudinal direction of the strip. Typically a contact element 3 comprises an elongate plate-like shape and advantageously the longitudinal extent of the contact element 3 is arranged transversely to the length of the strap 6 (as shown in figure 2). According to present invention preferably each contact element 3 is wetted with the preparation 4 as indicated by the dotted area.

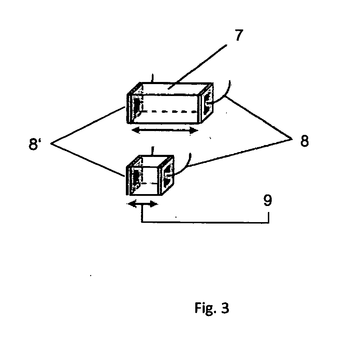

Resistance measurements of topical preparations may be conducted with an experimental setup as depicted in Fig. 3. The experimental setup consists essentially of a container of non conducting material (7). In said container electrodes (8, 8') of a defined surface are are arranged in a mutual position set apart in a defined distance (9). Preferably the electrodes are flat and arranged parallel, facing each other with their planes. Further in order to measure resistance and conductivity of a fluid, gel or cream, said substance is filled into the container (7), the electrodes are connected to a power source forming an electrical circuit and appropriate measurement instruments are connected to the circuit.

The described invention is useful to optimize the electrical properties of the contact between one or several electrodes, typically an array of electrodes, and skin in living beings, particularly humans.

While this invention is susceptible of embodiments in many different forms, there is described herein in detail, preferred embodiments of the invention with the understanding that the present disclosure is to be considered as an exemplification of the principles of the invention and is not intended to limit the broad aspects of the invention to the embodiments illustrated.

### EXAMPLES

Below presented experimental setup comprehends the general framework to determine the conductance (or resistance) of a material, e.g. such as a fluid, a gel, or a cream.

Small plastic cubes/cuboids (7) of 1 cm³ or 2 cm³ (see fig. 3) were filled with preparations according to present invention. Copper electrodes (8, 8') of 1 cm² were arranged to have a volume of 1 cm³ or 2 cm³ of preparation in between them at an electrode distance (9) of 1 cm or 2 cm, respectively. The resistance was measured with the programmable LCR - Bridge HM8118, by Hameg Instruments GmbH, Industriestrasse 6, D-63533 Mainhausen, Germany, at frequencies of 200 kHz, 100 kHz, or 50 kHz.

Tested preparations according to present disclosure comprise compositions within the following range of example 1, see table below. Most preferred compositions comprise values within the closer range presented in the third column of the table below.

| | Example 1 (in weight percentage) | preferred range of example 1 (in weight percentage) |
|---|---|---|
| Water | 50-80 | 55-77 |
| Oil/s | 20-45 | 20-40 |
| Alcohol/s | 1-20 | 4-15 |
| Additives | 0-5 | 0.5-4 |

The additives comprise skin compatible surfactants (surface active agents), optionally also humectants, odorants, and/or colorants etc.

Comparative examples were prepared from commercially available electrode creams and electrode sprays:
- Comparative example 1: from Sigma, electrode cream, REF 17-05, by Parker Laboratories, Inc.
- Comparative example 2: Dispo Contact, EKG-Elektrode Spray, Pharmacode 2817886.

Measured exemplary values of preparations are presented in the following table. The tested preparation of example 1 is particularly suited for EIT belt applications. The different frequencies used show a small effect only.

| | **200 kHz** | **100 kHz** | **50 kHz** |
|---|---|---|---|
| **Comparative example 1** | 15.6 mS/cm | 15.5 mS/cm | 15.4 mS/cm |
| **Comparative example 2** | 75.6 mS/cm | 74.1 mS/ cm | 77.5 mS/cm |
| **Example 1** | 0.253 mS/cm | 0.239 mS/cm | 0.227 mS/cm |

## Claims

1. Electrode sensor kit for establishing electrical contact with skin (2) comprising the following components:
- at least one contact element (3) connectable to an analytical instrument, and
- a preparation (4) comprising a mixture of water and at least one lipid for enhancing electrical contact properties between said contact element (3) and the skin (2) said mixture forming an emulsion, in particular a water-in-oil or an oil-in-water emulsion,
**characterized in that**
said preparation has a conductivity of less than 1 mS/cm.

2. Electrode sensor kit according to the preceding claim **characterised in that** said preparation (4) comprises at least an active ingredient selected from the group consisting of hygroscopic substances, hydrophilic substances, saccharides or polysaccharide, polyacrylates, panthenol or D-panthenol, allantoin, aloe vera, glycosaminoglycans, anionic nonsulfated glycosaminoglycans, algae or alginic acid, amino acids or proteins, and hyaluronic acid or salt; whereby hyaluronic acid is preferred.

3. Electrode sensor kit according to any of the preceding claims **characterised in that** said preparation (4) further comprises at least an alcohol, preferably an alcohol selected from the group consisting of mono-, di-, tri-, and polyhydroxy alcohols, glycerol, sorbitol, propylene glycol, and combinations thereof.

4. Electrode sensor kit according to any one of the preceding claims **characterised in that** said at least one lipid is selected from the group consisting of oils, preferably vegetable oils; phospholipids, preferably diacylphospholipids, phosphatidylcholine, phosphatidylethanolamine, phosphatidylglycerol, phosphatidylserine, phosphatidylinositol, and their monoacyl derivatives; cholesterol; natural lecithins, preferably natural lecithins from egg, milk, soy, sunflower, and/or oat; enzyme hydrolysed lecithins, preferably enzyme hydrolysed soy lecithins; mixtures of monoacylphospholipids and diacylphospholipids, said mixtures preferably containing 10 to 90 percent by weight of monoacylphospholipids; and combinations thereof.

5. Electrode sensor kit according to any one of the preceding claims **characterised in that** said at least one lipid and/or active ingredient is present in the form of lipo-somes or nano-particles.

6. Electrode sensor kit according to any one of the preceding claims **characterized in that** the preparation (4) is in the form of a fluid, a gel, or a cream.

7. Electrode sensor kit according to any one of the preceding claims **characterized in that** the amount of the at least one lipid in the preparation (4) is in the range of 5 to less than 50 weight percent, preferably in the range of 10 to 45 weight percent, and more preferably in the range of 15 to 40 percent.

8. Electrode sensor kit according to any one of the preceding claims **characterized in that** the amount of water in the preparation (4) is in the range of 50 to 90 weight percent, preferably in the range of 50 to 85 weight percent, and more preferably in the range 50 to 80 weight percent.

9. Electrode sensor kit according to any one of the preceding claims, **characterized in that** said at least one contact element (3) comprises a material selected from the group consisting of metals, conductive polymers, textiles and conductive textiles, or a combination thereof.

10. Electrode sensor kit according to any one of the preceding claims **characterized in that** the at least one contact element (3) comprises a structure or a layer of porous material on its skin contacting surface, which is wetted or impregnated with the preparation (4).

11. Electrode sensor kit according to claim 10 **characterized in that** the structured surface is uneven, pocketed and/or porous.

12. Electrode sensor kit according to any one of the preceding claims **characterized in that** the at least one contact element (3) comprises a material selected from the group consisting of metals, conductive polymers, textiles and conductive textiles, or a combination thereof.

13. Electrode sensor assembly (1) for establishing electrical contact with skin (2) comprising a plurality of contact elements (3) connectable to an analytical instrument, wherein each contact element comprises a surface for contacting the skin, said surface being coated or impregnated with a preparation (4) comprising a mixture of water and at least one lipid for enhancing electrical contact properties between said contact elements (3) and the skin (2), and said mixture forming an emulsion, in particular a water-in-oil or an oil-in-water emulsion
**characterized in that**
said preparation has a conductivity of less than 1 mS/cm.

## Patentansprüche

1. Sensorelektrodenset zur Herstellung elektrischen Kontaktes mit Haut (2), folgende Teile umfassend:
- mindestens ein Kontaktstück (3), das an ein Analyseinstrument angeschlossen werden kann, und
- eine Zubereitung (4), die ein Gemisch von Wasser mit mindestens einem Lipid umfasst, zur Verbesserung der elektrischen Kontakteigenschaften zwischen dem genannten Kontaktstück (3) und der Haut (2), wobei das genannte Gemisch eine Emulsion bildet, insbesondere eine Wasser-in-Öl- oder eine Ö-in-Wasser-Emulsion,
**dadurch gekennzeichnet, dass**
die genannte Zubereitung eine Leitfähigkeit von weniger als 1 mS/cm aufweist.

2. Sensorelektrodenset nach dem vorangehenden Patentanspruch, **dadurch gekennzeichnet, dass** die genannte Zubereitung (4) mindestens einen Wirkstoff umfasst, der aus der Gruppe, bestehend aus hygroskopischen Substanzen, hydrophilen Substanzen, Sacchariden und Polysacchariden, Polyacrylaten, Panthenol oder D-Panthenol, Allantoin, Aloe vera, Glykosaminoglykanen, anionischen, nicht sulphatierten Glykosaminoglykanen, Algen oder Alginsäure, Aminosäuren oder Proteinen und Hyaluronsäure oder Salz besteht, wobei Hyaluronsäure bevorzugt wird, ausgewählt wurde.

3. Sensorelektrodenset nach irgendeinem der vorangehenden Patentansprüche, **dadurch gekennzeichnet, dass** die genannte Zubereitung (4) außerdem mindestens einen Alkohol, vorzugsweise einen Alkohol aus der Gruppe, bestehend aus Mono-, Di-, Tri- und Polyalkoholen, Glyzerin, Sorbit, Propylenglykol und Gemischen davon, enthält.

4. Sensorelektrodenset nach irgendeinem der vorangehenden Patentansprüche, **dadurch gekennzeichnet, dass** das genannte mindestens eine Lipid aus der Gruppe, bestehend aus Ölen, vorzugsweise Pflanzenölen, Phospholipiden, vorzugsweise Diacylphopholipiden, Phophatidylcholin, Phophatidylethanolamin, Phophatidylglyzerin, Phophatidylserin, Phophatidylinositol und deren Monoacylderivaten; Cholesterin; natürlichen Lezithinen, vorzugsweise natürlichen Lezithinen aus Ei, Milch, Soya, Sonnenblumen und/oder Hafer; durch Enzyme hydrolysierten Lezithinen, vorzugsweise durch Enzyme hydrolysierten Soyalezithinen; Gemischen aus Monoacylphospholipiden und Diacylphospholipiden, wobei die genannten Gemische vorzugsweise 10 bis 90 Gewichtsprozent Monoacylphospholipide enthalten; und Gemischen davon, ausgewählt wurde.

5. Sensorelektrodenset nach irgendeinem der vorangehenden Patentansprüche, **dadurch gekennzeichnet, dass** das genannte mindestens eine Lipid und/oder Wirkstoff in Form von Liposomen oder Nanopartikeln vorliegt.

6. Sensorelektrodenset nach irgendeinem der vorangehenden Patentansprüche, **dadurch gekennzeichnet, dass** die Zubereitung (4) die Form einer Flüssigkeit, eines Gels oder einer Creme hat.

7. Sertsorelektrodenset nach irgendeinem der vorangehenden Patentansprüche, **dadurch gekennzeichnet, dass** die Menge des genannten mindestens einen Lipids in der Zubereitung (4) im Bereich von 5 bis weniger als 50 Gewichtsprozent, vorzugsweise im Bereich von 10 bis 45 Gewichtsprozent und stärker bevorzugt im Bereich von 15 bis 40 Prozent liegt.

8. Sensorelektrodenset nach irgendeinem der vorangehenden Patentansprüche, **dadurch gekennzeichnet, dass** der Wassergehalt der Zubereitung (4) im Bereich von 50 bis 90 Gewichtsprozent liegt, vorzugsweise im Bereich von 50 bis 85 Gewichtsprozent und stärker bevorzugt im Bereich von 50 bis 80 Gewichtsprozent.

9. Sensorelektrodenset nach irgendeinem der vorangehenden Patentansprüche, **dadurch gekennzeichnet, dass** das genannte mindestens eine Kontaktstück (3) einen Werkstoff aufweist, ausgewählt aus der Gruppe, bestehend aus Metallen, elektrisch leitenden Polymeren, Textilien und elektrisch leitenden Textilien, oder einer Kombination davon.

10. Sensorelektrodenset nach irgendeinem der vorangehenden Patentansprüche, **dadurch gekennzeichnet, dass** das genannte mindestens eine Kontaktstück (3) eine Struktur oder Schicht aus porösem Werkstoff auf seiner Hautkontaktseite aufweist, die mit der Zubereitung (43) angefeuchtet oder imprägniert wird.

11. Sensorelektrodenset nach Patentanspruch 10, **dadurch gekennzeichnet, dass** die strukturierte Oberfläche uneben, mit Vertiefungen versehen und/oder porös ist.

12. Sensorelektrodenset nach irgendeinem der vorangehenden Patentansprüche, **dadurch gekennzeichnet, dass** das genannte mindestens eine Kontaktstück (3) einen Werkstoff aufweist, ausgewählt aus der Gruppe, bestehend aus Metallen, elektrisch leitenden Polymeren, Textilien und elektrisch leitenden Textilien, oder einer Kombination davon.

13. Sensorelektrodeneinheit (1) zur Herstellung elektrischen Kontaktes mit Haut (2), mehrere Kontaktstücke (3) umfassend, die an ein Analyseinstrument angeschlossen werden können, wobei jedes Kontaktstück eine Oberfläche für den Kontakt mit der Haut aufweist, wobei die genannte Oberfläche mit einer Zubereitung (4) beschichtet oder imprägniert ist, die ein Gemisch aus Wasser und mindestens einem Lipid umfasst, um die elektrischen Kontakteigenschaften zwischen den genannten Kontaktstücken (3) und der Haut (2) zu verbessern, und wobei das genannte Gemisch eine Emulsion bildet, insbesondere eine Wasser-in-Öl-oder eine Öl-in-Wasser-Emulsion,
**dadurch gekennzeichnet, dass**
die genannte Zubereitung eine Leitfähigkeit von weniger als 1 mS/cm aufweist.

## Revendications

1. Kit de capteur à électrodes pour établir le contact électrique avec la peau (2) comprenant les composants suivants :
- au moins un élément de contact (3) qui peut être connecté à un instrument d'analyse et
- une préparation (4) qui comprend un mélange d'eau et d'au moins un lipide pour augmenter les propriétés de contact électrique entre ledit élément de contact (3) et la peau, ledit mélange formant une émulsion, en particulier une émulsion eau dans l'huile ou huile dans eau,
**caractérisé en ce que** ladite préparation a une conductivité de moins de 1 mS/cm.

2. Kit de capteur à électrodes selon la revendication précédente, **caractérisé en ce que** ladite préparation (4) comprend au moins un ingrédient actif sélectionné dans le groupe composé des substances hygroscopiques, des substances hydrophiles, des saccharides ou du polysaccharide, des polyacrylates, du panthénol ou du D panthénol, de l'allantoïne, de l'aloe vera, des glycosaminoglycanes, des glycosaminoglycanes anioniques non sulfatés, des algues ou de l'acide alginique, des aminoacides ou des protéines et de l'acide hyaluronique ou du sel, l'acide hyaluronique étant préféré.

3. Kit de capteur à électrodes selon l'une quelconque des revendications précédentes, **caractérisé en ce que** ladite préparation (4) comprend de plus au moins un alcool, de préférence un alcool sélectionné dans le groupe composé des monoalcools, des dialcools, des trialcools et des polyhydroxyalcools, du glycérol, du sorbitol, du propylène glycol et des combinaisons de ceux-ci.

4. Kit de capteur à électrodes selon l'une quelconque des revendications précédentes, **caractérisé en ce que** le lipide qui existe au moins est sélectionné dans le groupe composé des huiles, de préférence des huiles végétales, des phospholipides, de préférence des diacylphospholipides, de la phosphatidylcholine, de la phosphatidyléthanolamine, du phosphatidylglycérol, de la phosphatidylsérine, du phosphatidylinositol et de leur dérivés monoacyles, du cholestérol, des lécithines naturelles, de préférence les lécithines naturelles de l'oeuf, du lait, du soja, du tournesol et/ou de l'avoine, des lécithines hydrolysées par les enzymes, de préférence les lécithines de soja hydrolysées par les enzymes, des mélanges de monoacylphospholipides et des diacylphospholipides, lesdits mélanges contenant de préférence 10 à 90 pour-cent en poids de monoacylphospholipides et des combinaisons de ceux-ci.

5. Kit de capteur à électrodes selon l'une quelconque des revendications précédentes, **caractérisé en ce que** ledit lipide qui existe au moins et/ou ingrédient actif est présent sous forme de liposomes ou de nanoparticules.

6. Kit de capteur à électrodes selon l'une quelconque des revendications précédentes, **caractérisé en ce que** la préparation (4) est sous forme d'un fluide, d'un gel ou d'une crème.

7. Kit de capteur à électrodes selon l'une quelconque des revendications précédentes, **caractérisé en ce que** la quantité du lipide qui existe au moins dans la préparation (4) est de l'ordre de 5 à moins de 50 pour-cent en poids, de préférence de l'ordre de 10 à 45 pour-cent en poids et de manière plus préférée de l'ordre de 15 à 40 pour-cent.

8. Kit de capteur à électrodes selon l'une quelconque des revendications précédentes, **caractérisé en ce que** la quantité d'eau dans la préparation (4) est de l'ordre de 50 à 90 pour-cent en poids, de préférence de 50 à 85 pour-cent en poids et de manière plus préférée de l'ordre de 50 à 80 pour-cent.

9. Kit de capteur à électrodes selon l'une quelconque des revendications précédentes, **caractérisé en ce que** ledit élément de contact qui existe au moins (3) comprend un matériau sélectionné dans le groupe composé des métaux, des polymères conducteurs, des textiles et des textiles conducteurs ou d'une combinaison de ceux-ci.

10. Kit de capteur à électrodes selon l'une quelconque des revendications précédentes, **caractérisé en ce que** l'élément de contact qui existe au moins (3) comprend une structure ou une couche de matériau poreux sur sa surface qui est en contact avec la peau qui est mouillée ou imprégnée de la préparation (4).

11. Kit de capteur à électrodes selon la revendication 10, **caractérisé en ce que** la surface structure est inégale, avec des renfoncements et/ou poreuse.

12. Kit de capteur à électrodes selon l'une quelconque des revendications précédentes, **caractérisé en ce que** l'élément de contact qui existe au moins (3) comprend un matériau sélectionné dans le groupe composé des métaux, des polymères conducteurs, des textiles et des textiles conducteurs ou d'une combinaison de ceux-ci.

13. Ensemble de capteur à électrodes (1) pour établir le contact électrique avec la peau (2) comprenant une pluralité d'éléments de contact (3) qui peuvent être connectés à un instrument d'analyse, chaque élément de contact comprenant une surface pour être en contact avec la peau, ladite surface étant revêtue ou imprégnée d'une préparation (4) comprenant un mélange d'eau et d'au moins un lipide pour augmenter les propriétés de contact électrique entre lesdits éléments de contact (3) et la peau (2) et ledit mélange formant une émulsion, en particulier une émulsion eau dans l'huile ou huile dans eau, **caractérisé en ce que** ladite préparation a une conductivité de moins de 1 mS/cm.
